# EUROPEAN PATENT SPECIFICATION

(11) **EP 0 717 965 B1**
(45) Date of publication and mention of the grant of the patent: **13.10.1999**
(21) Application number: 95305456.6
(22) Date of filing: 04.08.1995
(51) Int. Cl.: A61B 17/36

(54) **Device for treating the interior of body cavities with laser energy**
Behandlungsvorrichtung für Körperhöhlen mit Laserenergie
Dispositif de traitement de l'intérieur de cavités du corps avec l'énergie d'un laser

(30) Priority: 21.12.1994 IL 11210794
(43) Date of publication of application: 26.06.1996
(73) Proprietor: LASER INDUSTRIES LIMITED, Tel Aviv 61131 (IL)
(72) Inventor: Konwitz, Ellie, 52 432 Ramat Gan (IL); Donnez Jacques, 1150 Brussels (BE)
(74) Representative: Gold, Tibor Z.

(56) References cited:
- WO-A-89/06935
- WO-A-94/00061
- WO-A-96/06574
- DE-A- 2 821 376
- US-A- 4 790 310
- US-A- 5 298 026
- US-A- 5 354 296
- US-A- 5 449 354

## Description

The present invention relates to a device for treating the interior of a body cavity with laser energy. The invention is particularly useful for the treatment of chronic menorrhagia, and is therefore described below with respect to such an application, but it will be appreciated that the invention could be used in other applications as well.

Chronic menorrhagia, defined as excessive and/or prolonged menstrual bleeding, is commonly treated by birth control pills, other hormonal therapies, or by a minor operation called "D and C" (dilation and curettage) involving a scraping of the lining of the uterus. When such treatments are not effective, a hysterectomy is generally performed involving removing the uterus and the lining along with it. Approximately 600,000 hysterectomies are performed in the USA each year.

Recently, a technique has been developed using laser energy to burn the uterine lining such as to cause scarring that prevents the lining from growing back. In this technique, a laser beam is conducted into the uterus by means of an optical fiber. The optical fiber is inserted via a channel of a hysteroscope, enabling the physician to view the interior of the uterus as the physician manipulates the tip of the optical fiber. The physician sweeps the tip of the optical fiber across the uterine lining to ablate the lining to a depth of about 3-5 mm. This procedure is generally quite painful because of the need to insert the hysteroscope and to dilate the uterus, usually done with a liquid, in order to enable the physician to view all the surfaces of the uterine lining as the optical fiber is manipulated over it.

WO-A-8906935 discloses a catheter having outwardly moveable optical fibers as claimed in the preamble of claims 1 and 4.

An object of the present invention is to provide generally a device for treating the interior of a body cavity with laser energy, and particularly a device for treating chronic menorrhagia with laser energy in order to ablate or coagulate the uterine lining.

According to the present invention, there is provided a device for treating the interior of a body cavity with laser energy as claimed in claims 1 and 4.

According to the described embodiments, the actuator means comprises a mechanical coupling between the distal ends of the fibers effective, when the proximal end of the first fiber is manually moved axially with respect to that of the second fiber, to move the distal ends of the fibers to the spread-apart relation.

Such a laser device is particularly useful for treating chronic menorrhagia since a single dosage of laser energy will substantially cover most or all of the uterine lining. Thus, the device avoids the need for the physician to view the interior of the uterus, and thereby the need for inserting a hysteroscope into the uterus. It also reduces, or perhaps may even eliminate, the dilation required of the uterus, and the time of treatment.
Fig. 1 illustrates one form of device constructed in accordance with the present invention, the device being shown in its initial, non-operative condition;
Fig. 2 is a view of the device of Fig. 1 in its operative condition.
Fig. 3 is a cross-sectional view along line III--III of Fig. 2;
Figs. 4a-4f illustrate the use of the device of Figs. 1 and 2 in treating chronic menorrhagia;
Fig. 5 is a plan view illustrating a second form of device constructed in accordance with the present invention, the device being shown in its initial, non-operative condition;
Figs. 5a, 5b and 5c are enlarged sectional views, along lines 5a--5a, 5b--5b and 5c--5c, respectively, of Fig. 5;
Fig. 6 is a plan view illustrating the device of Fig. 5 in its operative condition;
Figs. 7a, 7b and 7c are end, plan and sectional views, respectively, more particularly illustrating the construction of the stiffening spine in the device of Figs. 5 and 6;
Fig. 8 is an enlarged sectional view illustrating the coupling structure at the distal end of the device;
Figs. 8a, 8b and 8c are fragmentary views illustrating the manner of making the coupling structure of Fig. 8;
Fig. 9 illustrates the condition of the distal end of the device at the time of insertion into the body cavity to be treated; and
Fig. 10 illustrates the condition of the distal end of the device after insertion into the body cavity and at the time of treatment thereof by laser radiation.

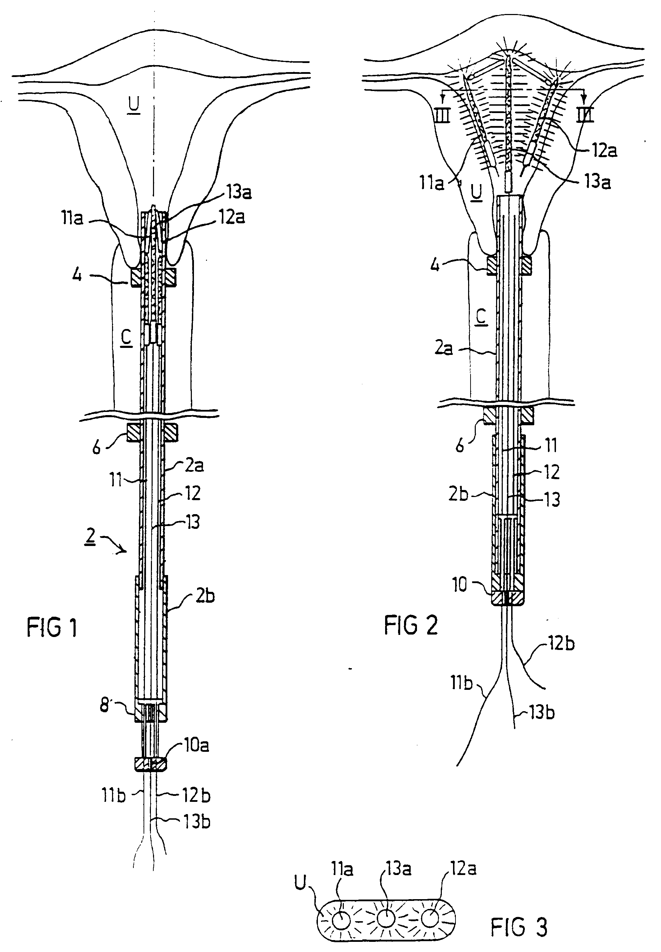

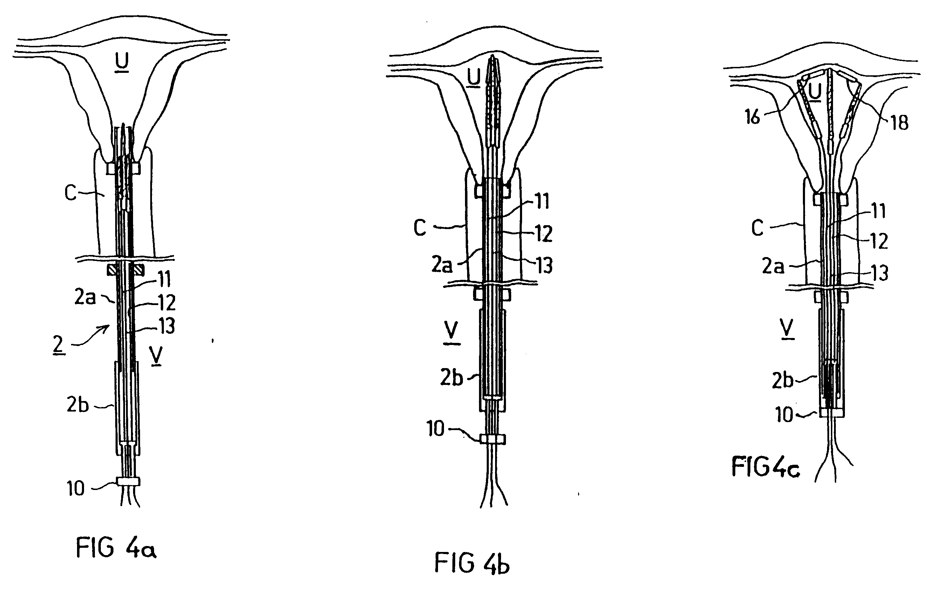

The device illustrated in Figs. 1-4f comprises a cannula, generally designed 2, constituted of two telescoping sections, namely an outer or distal section 2a, and an inner or proximal section 2b. When the device is used for treating chronic menorrhagia, as shown in Figs. 4a-4f, the distal section 2a of the cannula is inserted via the patient's vagina V and cervix C into the uterus U; whereas the outer end of the proximal section 2b of the cannula is disposed externally of the patient's vagina. The two telescoping section 2a, 2b of the cannula are flattened to correspond to the flattened shape of the vagina as shown in Fig. 3. A ring 4 fixed to the distal end of cannula section 2a limits against the mouth of the uterus U during use of the device, and another ring 6 fixed to a mid-portion of cannula section 2a limits against the mouth of the cervix C. The distal end of cannula section 2a is open, whereas the proximal end of cannula section 2b is closed by an end wall 8 penetrated by a plunger 10 movable within cannula section 2b.

Three optical fibers 11, 12 and 13, are disposed in side-by-side relation within cannula 2. Each optical fiber includes a distal end 11a, 12a, 13a, to be located within the uterus during use of the device, and a proximal end 11b, 12b, 13b, to be located externally of the patient's body and to be coupled to a source of laser energy. The two outer optical fibers 11, 12 are axially movable with respect to the middle optical fiber 13. For this purpose, the proximal ends of the two outer fibers 11, 12 are fixed to the plunger 10, whereas the middle fiber 13 passes through a bore 10a in plunger 10 so that movement of the plunger into cannula section 2b will move the two outer fibers 11, 12 axially with respect to the middle fiber 13. The middle fiber 13, however, is fixed to the end wall 8 of cannula section 2b so that cannula section 2b may be moved, together with plunger 10, to move all three optical fibers axially within the cannula.

The distal ends 11a, 12a, 13a, of the three optical fibers are roughened on their outer surfaces so that the laser energy, applied to their proximal ends 11b, 12b, 13b, is conducted through the fibers and is scattered laterally outwardly at their distal ends.

As shown particularly in Fig. 2, the distal ends 11a, 12a, 13a, of the three optical fibers are coupled together such that the movement of the two outer optical fibers 11, 12, axially with respect to the middle fiber 13, causes the distal ends of the two outer fibers to spread apart laterally. For this purpose, the distal ends of the outer fiber 11 and the middle fiber 13 are coupled together by a rod 16 pivotally mounted at its opposite ends to the two fibers; similarly, the distal ends of the outer fiber 12 and middle fiber 13 are coupled together by a second rod 18 pivotally mounted at its opposite ends to the two fibers.

Preferably, the roughened distal ends 11a, 12a, 13a of the three optical fibers are each covered by an outer glass or quartz tube to avoid carbonization thereon. In addition, the rods 16, 18 are preferably of metal.

Figs. 4a-4f illustrate one manner of using the above-described device for treating chronic menorrhagia.

Initially, the illustrated device is in the condition illustrated in Fig. 4a (also in Fig. 1), wherein cannula section 2b is moved outwardly so as to move all three optical fibers 11, 12, 13, to their retracted, non-operative positions, wherein the fibers, and particularly their distal ends, are completely enclosed by the cannula 2. In this initial position, the plunger 10 is also in its outer position such that rods 16, 18 cause the distal tips of the three fibers to assume a substantially parallel, side-by-side relation to each other.

The cannula is then inserted through the patient's vagina V and cervix C to the position illustrated in Fig. 4a, wherein ring 4 abuts against the mouth of the uterus U, ring 6 abuts against the mouth of the cervix C, and a portion of the proximal cannula section 2b extends externally of the patient's vagina. Cannula section 2b is then moved axially with respect to cannula section 2a to the position illustrated in Fig. 4b. This causes the distal ends of the three fibers to move into the patient's uterus U. Plunger 10 is then moved axially with respect to cannula section 2b, which causes the two optical fibers 11, 12, to move axially with respect to the middle fiber 13, as shown in Fig. 4c (and also in Fig. 2). During this movement of the two outer fibers 11, 12, the rods 16, 18, connecting their distal ends to the middle fiber 13, cause the distal ends of the outer fibers to spread laterally with respect to the middle fiber.

When the device is in this condition, laser energy is applied for a predetermined time period to the proximal ends of the three optical fibers 11, 12, 13. This laser energy is transmitted through each of the fibers to their respective distal ends 11a, 12a, 13a, and is scattered laterally outwardly of the optical fibers by the roughened surfaces at these distal ends. Since the uterus U is relatively flat as shown in Fig. 3, substantially the complete uterine lining is thus exposed to the laser energy without the need to rotate or otherwise manipulate the optical fibers in order to cover the complete surface of the uterine lining.

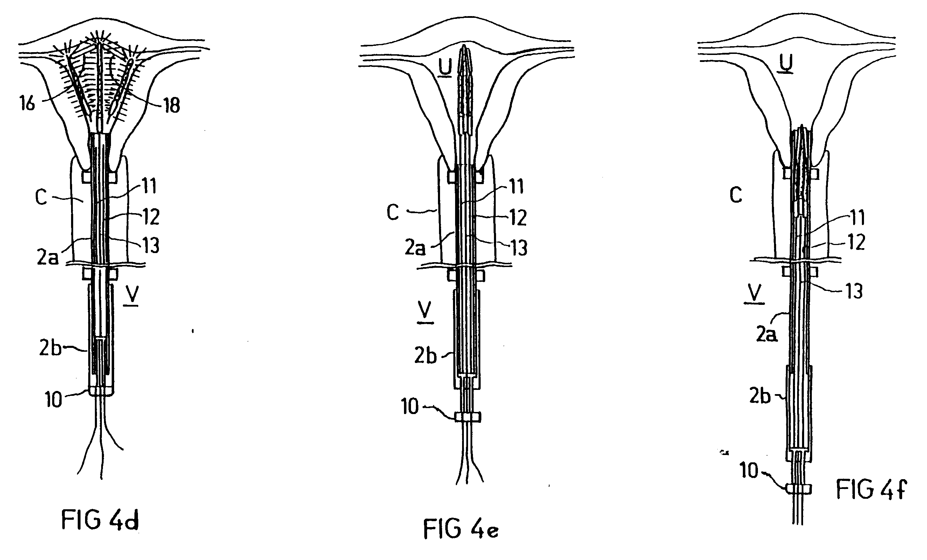

After this exposure of the uterine lining to the laser energy, the cannula 2 is pulled about 1.5 cm out of the cervix C (Fig. 4d). The plunger 10 is then withdrawn from cannula section 2b to thereby pivot the distal ends 11a, 12a, of the two outer fibers back to their side-by-side positions with respect to the distal end 13a of the middle fiber (Fig. 4e). Cannula section 2b is then moved outwardly with respect to cannula section 2a to thereby move all three fibers to their retracted, non-operative positions (Fig. 4f). The cannula may then be withdrawn.

For purposes of example, the roughened distal ends 11a, 12a, 13a, of the three optical fibers may be for a length of 30 mm; the laser energy applied may be that from a Nd-YAG laser; 10 watts may be applied to each optical fiber; and the exposure may be for a duration of about five minutes. Such a treatment causes ablation of the uterine lining to a depth of about 5 mm, which normally would be sufficient to prevent the uterine lining from growing back.

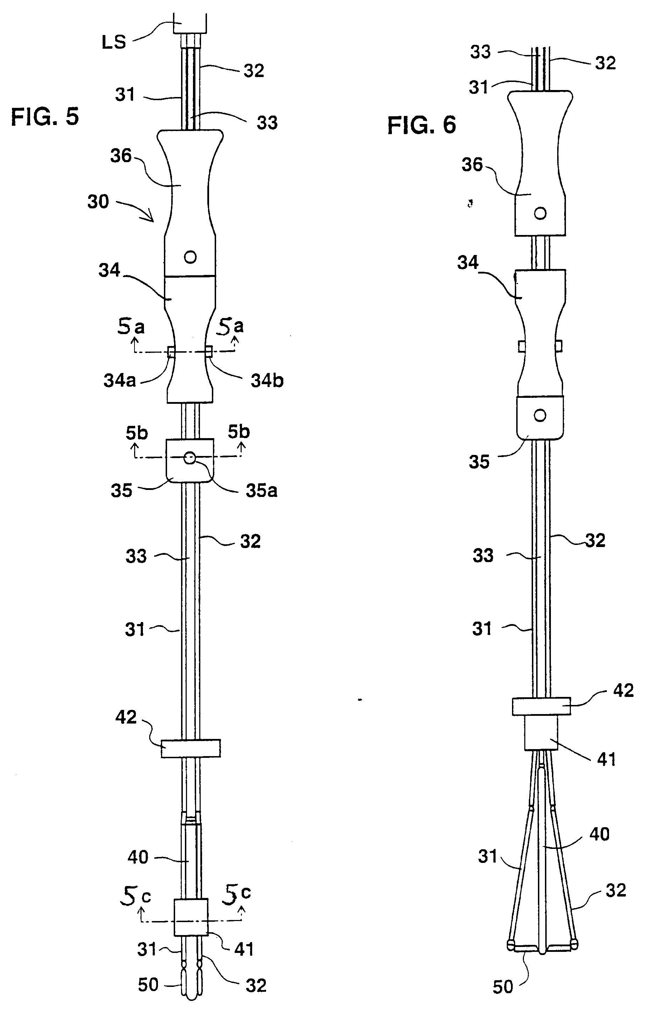

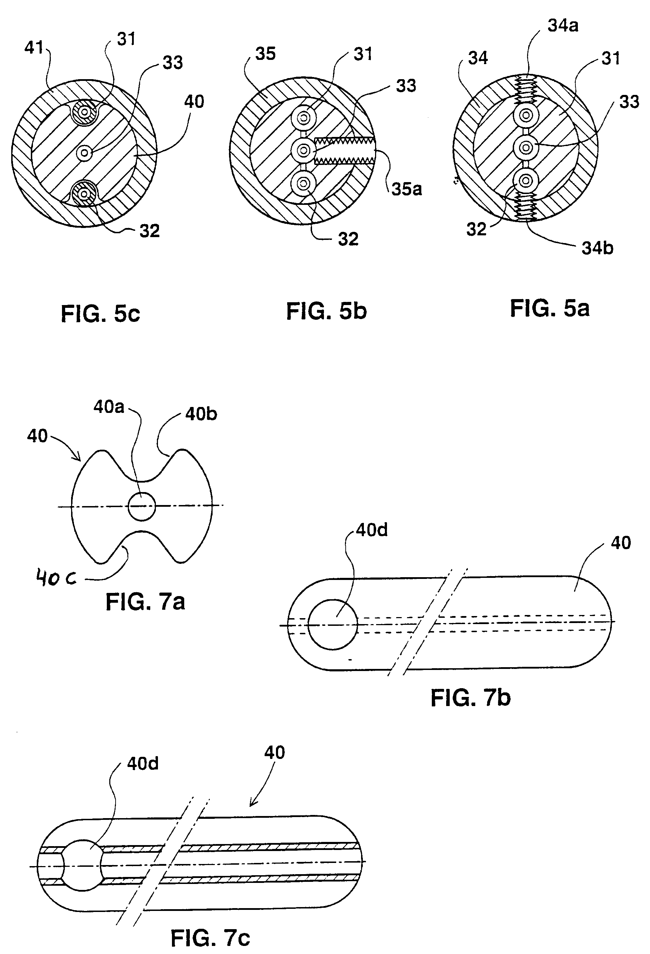

The device 30 illustrated in Figs. 5-10 is also useful for treating chronic menorrhagia by laser energy. It includes three optical fibers 31, 32, 33 in side-by-side relation and having distal ends to be inserted via the patient's vagina and cervix into the uterus, with the opposite, proximal ends to be located externally of the body and to be coupled to a source of laser energy, shown schematically as LS. The device further includes, at the proximal end of the three fibers, a handgrip 34 which is fixed to the two outer fibers 31, 32, as shown by set screws 34a, 34b, and a further handgrip 35 spaced towards the distal ends of the fibers and fixed by a set screw 35a to the middle fiber 33.

As will be described more particularly below, when handgrip 34 is moved with respect to handgrip 35, the two outer fibers 31, 32, are moved axially with respect to the inner fiber 33. This causes the distal ends of the two outer fibers to spread laterally with respect to the middle fiber 33 in order to spread the laser radiation over a relatively large surface, e.g., the lining of the uterus in the described application.

The proximal end of the device 30 further includes a rear stop 36 fixed to all three optical fibers and engageable with handgrip 34 to define the normal non-operative position of the handgrip as shown in Fig. 5. Handgrip 35 defines the operative position of handgrip 34 when the latter handgrip is moved to its operative position as shown in Fig. 6.

The construction and operation of the device of Figs. 5 and 6, insofar as described above, are generally similar to the device of Figs. 1-4f except that, instead of including a cannula enclosing the three optical fibers for retaining them in side-by-side relation, the device illustrated in Figs. 5-10 omits such a cannula. Rather, it includes a stiffening spine, generally designated 40.

Stiffening spine 40 is formed with a longitudinally-extending bore 40a for receiving the distal end of the middle fiber 33. It is also formed with two longitudinally-extending recesses 40b, 40c on its opposite sides for receiving and partially enclosing the distal ends of the two outer fibers 31, 32. The arrangement is such that the stiffening spine 40 normally supports the distal ends of the two outer fibers 31, 32 with respect to the middle fiber 33, but permits the distal ends of the two outer fibers to spread laterally away from the middle fiber when the two outer fibers are moved axially with respect to the middle fiber.

Stiffening spine 40 is further formed with a transverse opening 40d adjacent to its distal tip. Opening 40d receives a coupling member 50 which couples the distal tips of the two outer fibers 31, 32 to the distal tip of the middle fiber 33 to produce the above-described lateral spreading of the two outer fibers when they are moved axially to their extended, operative positions.

The three fibers 31-33 may be of a conventional construction, to include a core of silica, a plastic cladding, a plastic jacket, and a metal sheath. The metal sheath of the two outer fibers 31, 32 is removed from the portions of the fibers extending just past handgrip 35 to the distal ends of the fibers. The metal sheath of the middle fiber 33, however, is retained up to the connection to the stiffening spine 40 enclosing the middle fiber. The distal ends of all three fibers coextensive with the stiffening spine 40 are each covered by an outer glass or quartz tube 31a, 32a, and 33a, respectively. The outer surface of each glass tube is roughened for scattering the laser energy laterally outwardly of the optical fibers.

The distal ends of the three fibers 31-33 are enclosed by a retainer ring 41 when the fibers are in their retracted, non-operative position. When the device is to be actuated to its extended, operative position, retainer ring 41 is manually moved against a limit ring 42 fixed to the middle optical fibers 33, to permit the distal ends of the two outer fibers 31, 32, to spread laterally with respect to the stiffening spine 40 and the middle fiber 33.

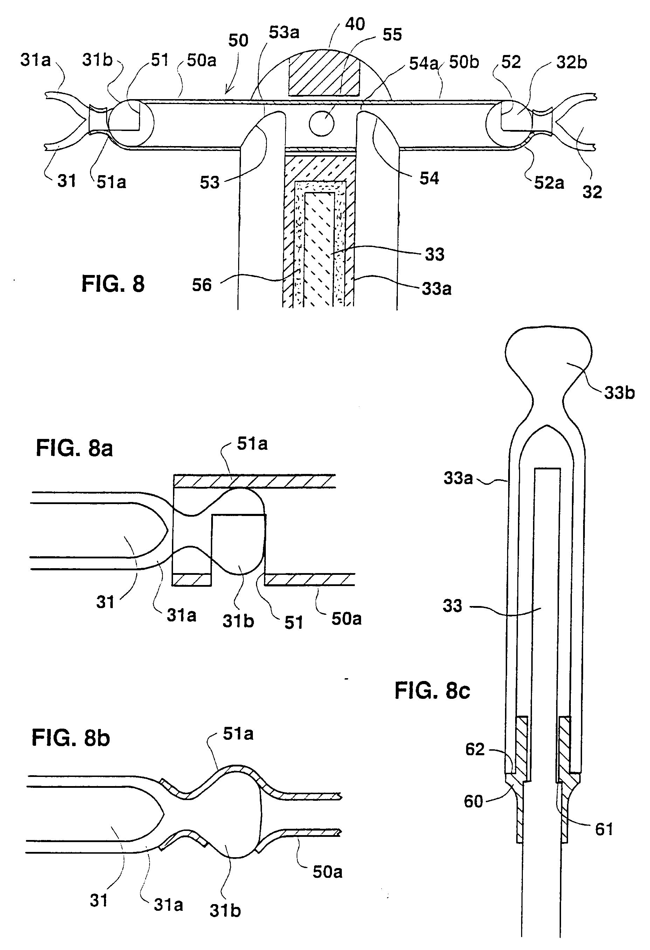

The lateral spreading of the distal ends of the two outer fibers 31, 32, by their axial movement with respect to the middle fiber 33, is effected by a coupling tube 50 (Fig. 8) of relatively stiff plastic material received exactly midway of its length within opening 40d of stiffening spine 40. One end of tube 50 receives the distal tip of outer fiber 31, and the opposite end of tube 50 receives the distal tip of outer fiber 32.

As shown in Fig. 8, tube 50 is initially formed with four notches 51-54. Notches 51 and 52 are adjacent to the outer ends of the tube 50 and define relatively narrow webs 51a, 52a, respectively, which serve as integral hinges permitting the ends of the tube outwardly of these notches to be easily pivotted with respect to the remainder of the tube. The other two notches 53, 54 are formed on opposite sides of the coupling of tube 50 to the stiffening spine 40. Notches 53, 54 define relatively narrow webs 53a, 54a, respectively, on opposite sides of the stiffening spine 40 and serve as integral hinges permitting the tube to freely bend at these webs also.

Tube 50 is preferably of "Teflon" (Reg. TM), which is a heat-shrinkable plastic. The outer glass or quartz tubes 31a-33a covering the distal ends of the three optical fibers 31-33 are formed with bulbous tips 31b-33b, respectively. Bulbous tips 31b and 32b, of the outer fibers 31 and 32 are inserted into the opposite ends of tube 50, after the above-described notches 51-54 have been formed. The respective ends of the tube are then heated, causing their ends to shrink from the initial condition (Fig. 8a) to the shrunken condition (Fig. 8b). This firmly secures the distal ends of the outer optical fibers 31, 32 to tube 50, while at the same time provides the thin webs 51a, 52a permitting the fiber ends to easily pivot with respect to the tube.

The middle fiber 33 is secured to its outer glass or quartz tube 33a by means of a metal ferrule 60 (Fig. 8c). The metal sheath of the middle fiber 33 is retained up to ferrule 60 and limits against an annular shoulder 61 formed in the ferrule. The outer glass or quartz tube 33a is received over the outer surface of ferrule 60 and limits against an outer annular shoulder 62. The bulbous tip 33b of tube 33a covering the middle fiber 33 is fixed within bore 40a of the stiffening spine 40 in any suitable manner, e.g., by adhesive. Tube 50 is fixed within opening 40d of the stiffening spine 40 in any suitable manner, e.g., by adhesive and/or by pin 55 passing through the end of the stiffening spine and a hole in tube 50.

As shown in Fig. 8, the optical fiber 33 is bonded to its outer glass or quartz tube 33a by a transparent adhesive 56. Fibers 31 and 32 may be bonded to their respective glass or quartz tubes 31a, 32a, in a similar manner.

It will thus be seen that tube 50 defines arms 50a, 50b on its opposite sides of equal length coupling the two outer fibers 31, 32 to the stiffening spine 40 fixed to the middle fiber 33, such that when the two outer fibers are axially moved with respect to the middle fiber, the distal ends of the two outer fibers spread apart from the middle fiber as shown in Fig. 6.

The device illustrated in Figs. 5-10 is used in the following manner:

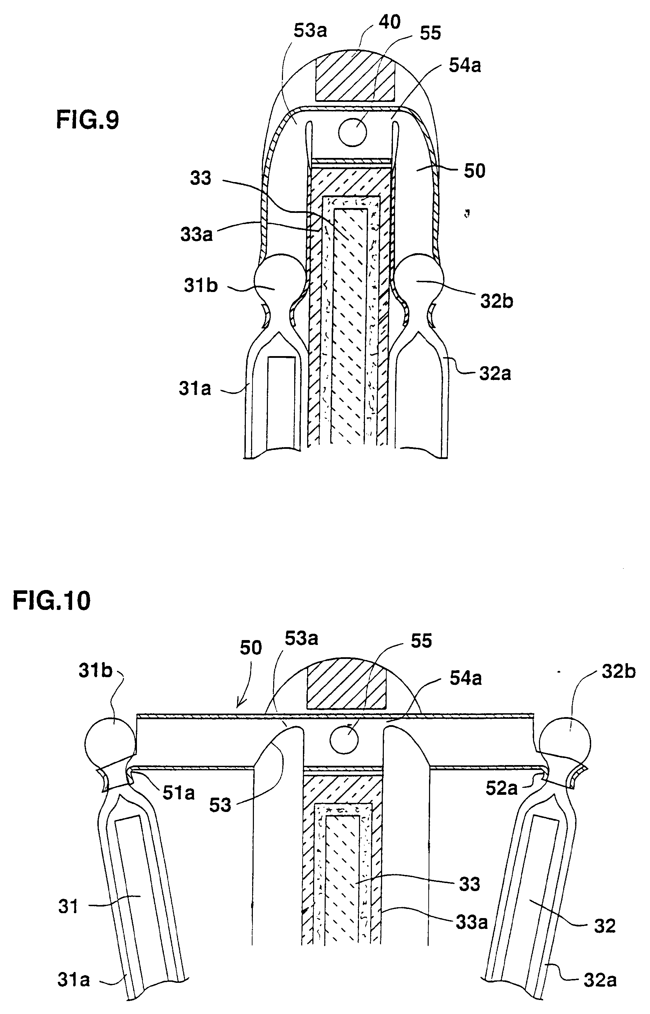

Limit ring 42 may first be preset according to the depth of insertion of the distal end of the device. With handgrip 34 abutting against the rear stop 36, and retainer ring 41 in its extended position (as shown in Fig. 5), the distal end of the device is inserted into the patient's vagina and cervix. At the time of insertion, the distal ends of the two outer fibers 31, 32, are in their retracted condition, parallel to the distal end of the middle fiber 33, as shown in Fig. 9.

Retainer ring 41 is then moved rightwardly against the stop ring 42, and handgrip 34 is moved leftwardly until it engages handgrip 35, as shown in Fig. 6. This movement causes the two outer fibers 31, 32 to move axially (leftwardly) with respect to the middle fiber 33, whereby coupling member 50 at the distal ends of the three fibers causes the two outer fibers to spread laterally with respect to the middle fiber. This is the operative condition of the distal ends of the fibers as illustrated in Fig. 10. The laser energy is then applied for a predetermined time to the proximal ends of the three fibers and is transmitted to the respective distal ends, where it is scattered laterally outwardly of the fibers by the roughened outer surfaces of the distal ends of the fibers.

While the invention has been described with respect to two preferred embodiments, it will be appreciated that these are set forth merely for purposes of example, and that many variations may be made. For example, the three optical fibers may be separately energized by an individual laser, or they may be all energized by a single laser outputting a single beam which is split into three beams by a beam splitter before applied to the proximal ends of the three fibers. A larger number of optical fibers could be used according to the nature of the surface to be radiated. The stiffening spine may be the outer covering of one of the fibers. Many other variations within the scope of the claims will be apparent.

## Claims

1. A device for treating the interior of a body cavity with laser energy, comprising at least two outer optical fibers (11,12), in side-by-side relation each having a distal end (11a,12a,) to be inserted into the body cavity to be treated, and a proximal end (11b,12b,) to be located externally of the body cavity and to be coupled to a source of laser energy; and actuator means (16, 18) coupled to said optical fibers (11,12,) for moving the distal ends of the fibers to a spread-apart relation; characterised by further comprising a middle optical fiber (13) inbetween said at least two outer optical fibers (11, 12), having a distal end to be inserted into the body cavity to be treated and a proximal end (13b) to be located externally of the body cavity and to be coupled to a source of laser energy, said actuator means comprising first and second rods (16,18) for coupling each of said at least two outer fibers (11,12) to said middle fiber (13), one end of each of said first and second rods (16,18) being pivotally connected to each of said distal ends (11a, 12a) of said outer fibers (11,12) and the other end of said first and second rods (16,18), being pivotally connected to said middle fiber (13), whereby by manually activating said proximal end of said outer fibers (11b, 12b) to move axially with respect to that of said middle fiber (13), the distal ends of the fibers (11a, 12a, 13a) are moved to a spread-apart relation.

2. The device according to claim 1, wherein said optical fibers are disposed within a cannula having a distal end adapted to be inserted into the body cavity to be treated, and a proximal end to be located externally of the body cavity when the distal end is inserted therein.

3. The device according to claim 2, wherein said proximal section of the cannula is telescopingly movable with respect to said distal section of the cannula; said fibers being fixed to said proximal section of the cannula to enable them to be manually moved axially together with respect to the distal section of the cannula.

4. A device for treating the interior of a body cavity with laser energy, comprising at least two outer optical fibers (31, 32) in side-by-side relation each having a distal end (31a, 32a) to be inserted into the body cavity to be treated and a proximal end to be located externally of the body cavity and to be coupled to a source of laser energy; and actuator means coupled to said fibers for moving the distal ends of said fibers to a spread-apart relation; and a stiffening spine (40) for normally supporting the distal ends of said outer fibers (31, 32), but permitting the distal end of at least one outer fiber to move laterally with respect to another outer fiber; characterised by further comprising a middle optical fiber (33) inbetween said at least two outer optical fibers (31, 32), having a distal end (33a) to be inserted into the body cavity to be treated and a proximal end to be located externally of the body cavity and to be coupled to a source of laser energy, and a coupling member (50) attached at its central region to the middle fiber (33) and at its opposite ends to the distal ends of the two outer fibers (31, 32); and wherein said stiffening spine (40) is formed with a longitudinally-extending bore (40a) for receiving the distal end of the middle fiber and is formed with a longitudinally-extending recess (40b, 40c) on each of its opposite sides for receiving and partially enclosing, at least portions of the distal ends of said outer fibers (31, 32); the two outer fibers (31, 32) being axially movable with respect to said middle fiber (33) and said stiffening spine (40), to move the distal ends of the outer fibers (31, 32) to a spread-apart relation.

5. The device according to any one of claims 1 - 4, wherein the device further includes a retainer ring enclosing the distal ends of said fibers normally to retain said distal ends in side-by-side relation, said retainer ring being movable towards the proximal ends of the fibers to permit said fibers to move laterally apart.

6. The device according to claim 5, wherein the device further includes a presettable limit ring to limit the movement of said retainer ring.

## Patentansprüche

1. Vorrichtung zur Behandlung des Inneren von Körperhöhlen mit Laserenergie, umfassend mindestens zwei nebeneinander angeordnete optische Fasern (11, 12), wobei jede ein distales Ende (11a, 12a) aufweist, zum Einführen in die zu behandelnde Körperhöhle, und ein nahegelegenes Ende (11b, 12b) zum Anordnen außerhalb der Körperhöhle und zum Verbinden mit einer Laserenergiequelle; und Betätigungsmittel (16, 18), welche mit den optischen Fasern (11, 12) verbunden sind, um die distalen Enden der Fasern voneinander abzuspreizen; **dadurch gekennzeichnet,** daß zusätzlich eine mittlere optische Faser (13) vorgesehen ist, welche zwischen den mindestens zwei äußeren Fasern (11, 12) angeordnet ist, mit einem distalen Ende zum Einführen in die zu behandelnde Körperhöhle und einem nahegelegenen Ende (13b) zum Anordnen außerhalb der Körperhöhle und zum Verbinden mit einer Laserenergiequelle, wobei die Betätigungsmittel ein erstes und ein zweites Stäbchen (16, 18) umfassen, welche jeweils die mindestens zwei äußeren Fasern (11, 12) mit der mittleren Faser (13) verbinden, wobei jeweils ein Ende des ersten und des zweiten Stäbchens (16, 18) schwenkbar mit den distalen Enden (11a, 12a) der äußeren Fasern (11, 12) verbunden ist und das jeweils andere Ende des ersten und des zweiten Stäbchens (16, 18) mit der mittleren Faser (13) schwenkbar verbunden ist, so daß durch manuelle Betätigung der nahegelegenen Enden der äußeren Fasern (11b, 12b), um diese axial gegenüber der mittleren Faser (13) zu bewegen, die distalen Enden der Fasern (11a, 12a, 13a) voneinander abgespreizt werden.

2. Vorrichtung nach Anspruch 1, bei welcher die optischen Fasern in einem Röhrchen angeordnet sind, mit einem distalen Ende, welches zum Einführen in die zu behandelnde Körperhöhle vorgesehen ist, und einem nahegelegenen Ende, welches außerhalb der Körperhöhle angeordnet ist, wenn das distale Ende in diese eingeführt ist.

3. Vorrichtung nach Anspruch 2, bei welcher der nahegelegene Abschnitt des Röhrchens teleskopartig gegenüber dem distalen Abschnitt des Röhrchens bewegbar ist, wobei die Fasern an dem nahegelegenen Abschnitt des Röhrchens befestigt sind, damit sie von Hand axial zusammen in Bezug zum distalen Abschnitt des Röhrchens bewegt werden können.

4. Vorrichtung zur Behandlung des Inneren von Körperhöhlen mit Laserenergie, umfassend zumindest zwei äußere optische Fasern (31, 32), welche nebeneinanderliegend angeordet sind, wobei jede ein distales Ende (31a, 32a) aufweist, zum Einführen in die zu behandelnde Körperhöhle, und ein nahegelegenes Ende aufweist, zum Anordnen außerhalb der Körperhöhle und zum Verbinden mit einer Laserenergiequelle; und Betätigungsmittel, welche mit den Fasern verbunden sind, um die distalen Enden der Fasern voneinander abzuspreizen; und einen Stützdorn (40), welcher die distalen Enden der äußeren Fasern (31, 32) seitlich abstützt, aber dabei dem distalen Ende mindestens einer äußeren Faser erlaubt, sich gegenüber einer anderen äußeren Faser seitlich nach außen zu bewegen, **dadurch gekennzeichnet**, daß sie ferner umfaßt: eine mittlere Faser (33) zwischen den mindestens zwei äußeren Fasern (31, 32), mit einem distalen Ende (33a) zum Einführen in die zu behandelnde Körperhöhle, und einem nahegelegenen Ende zum Anordnen außerhalb der Körperhöhle und zum Verbinden mit einer Laserenergiequelle, und ein Verbindungselement (50), dessen zentraler Abschnitt mit der mittleren Faser (33) und dessen gegenüberliegende Enden mit den distalen Enden der beiden äußeren Fasern (31, 32) verbunden sind, wobei der Stützdorn (40) eine langgezogene Bohrung (40a) zur Aufnahme des distalen Endes der mittleren Faser und eine langgezogene Ausnehmung (40b, 40c) auf jeder seiner gegenüberliegenden Seiten zur Aufnahme und teilweisen Umschließung zumindest eines Teils der distalen Enden der äußeren Fasern (31, 32) aufweist; wobei die beiden äußeren Fasern (31, 32) axial beweglich in Bezug zur mittleren Faser (33) und zum Stützdorn (40) ausgebildet sind, um die distalen Enden der äußeren Fasern (31, 32) voneinander abzuspreizen.

5. Vorrichtung nach einem der Ansprüche 1 bis 4, ferner umfassend einen Stützring, welcher normalerweise die distalen Enden der Fasern umgibt, um die distalen Enden seitlich nebeneinander liegend zu halten, wobei dieser Stützring beweglich gegenüber den nahegelegenen Enden der Fasern ausgeführt ist, um den Fasern eine seitliche Bewegung voneinander weg zu ermöglichen.

6. Vorrichtung nach Anspruch 5, ferner umfassend einen voreinstellbaren Begrenzungsring zur Begrenzung der Bewegung des Stützrings.

## Revendications

1. Dispositif pour traiter l'intérieur d'une cavité corporelle au moyen d'énergie laser, comprenant au moins deux fibres optiques extérieures (11,12), en relation côte à côte, et dont chacune a une extrémité distale (11a, 12a) adaptée à être insérée dans la cavité corporelle à traiter, et une extrémité proximale (11b, 12b) adaptée à être située à l'extérieur de la cavité corporelle et à être accouplée à une source d'énergie laser ; et des moyens d'actionnement (16,18) accouplés auxdites fibres optiques (11,12) pour amener les extrémités distales des fibres en une relation déployée, caractérisé en ce qu'il comprend, en outre, une fibre optique médiane (13) entre lesdites au moins deux fibres optiques extérieures (11,12), ayant une extrémité distale adaptée à être insérée dans la cavité corporelle à traiter et une extrémité proximale (13b) adaptée à être située à l'extérieur de la cavité corporelle et à être accouplée à une source d'énergie laser, lesdits moyens d'actionnement comprenant des première et seconde tiges (16,18) pour accoupler chacune desdites au moins deux fibres extérieures (11,12) à ladite fibre médiane (13), une extrémité de chacune desdites première et seconde tiges (16,18) étant connectée de façon pivotante sur chacune desdites extrémités distales (11a,12a) desdites fibres extérieures (11,12), et l'autre extrémité desdites première et seconde tiges (16,18) étant connectée de façon pivotante à ladite fibre médiane (13), grâce à quoi en actionnant manuellement de ladite extrémité proximale (11b,12b) desdites fibres extérieures pour les déplacer axialement par rapport à celles de ladite fibre médiane (13), les extrémités distales des fibres (11a,12a,13a) sont amenées en une relation déployée.

2. Dispositif selon la revendication 1, dans lequel lesdites fibres optiques sont disposées au sein d'une canule ayant une extrémité distale adaptée à être insérée dans la cavité corporelle à traiter, et une extrémité proximale adaptée à être située à l'extérieur de la cavité corporelle lorsque l'extrémité distale y est insérée.

3. Dispositif selon la revendication 2, dans lequel ladite section proximale de la canule est mobile télescopiquement par rapport à ladite section distale de la canule ; lesdites fibres étant fixées à ladite section proximale de la canule pour leur permettre d'être déplacées manuellement, axialement et ensemble par rapport à la section distale de la canule.

4. Dispositif pour traiter l'intérieur d'une cavité corporelle à l'aide d'énergie laser, comprenant au moins deux fibres optiques extérieures (31,32) en relation côte à côte, chacune ayant une extrémité distale (31a,32a) adaptée à être insérée dans la cavité corporelle à traiter et une extrémité proximale adaptée à être située à l'extérieur de la cavité corporelle et à être accouplée à une source d'énergie laser ; et des moyens d'actionnement accouplés auxdites fibres pour amener les extrémités distales desdites fibres en une relation déployée ; et une colonne formant raidisseur (40) pour supporter normalement les extrémités distales desdites fibres extérieures (31,32) tout en permettant à l'extrémité distale d'au moins une fibre extérieure de se déplacer latéralement par rapport à une autre fibre extérieure ; caractérisé en ce qu'il comprend, en outre, une fibre optique médiane (33) entre lesdites au moins deux fibres optiques extérieures (31,32), ayant une extrémité distale (33a) adaptée à être insérée dans la cavité corporelle à traiter et une extrémité proximale adaptée à être située à l'extérieur de la cavité corporelle et à être accouplée à une source d'énergie laser, et un élément d'accouplement (50) fixé au niveau de sa région centrale à la fibre médiane (33) et au niveau de ses extrémités opposées aux extrémités distales des deux fibres extérieures (31,32) ; et dans lequel ladite colonne formant raidisseur (40) comporte un perçage longitudinal (40a) pour recevoir l'extrémité distale de la fibre médiane et un retrait s'étendant longitudinalement (40b,40c) sur chacun de ses côtés opposés pour recevoir et enfermer partiellement au moins des portions des extrémités distales desdites fibres extérieures (31,32) ; les deux fibres extérieures (31,32) étant déplaçables axialement par rapport à ladite fibre médiane (33) et à ladite colonne formant raidisseur (40), pour amener les extrémités distales des fibres extérieures (31,32) en une relation déployée.

5. Dispositif selon l'une quelconque des revendications 1 à 4, dans lequel le dispositif comprend en outre une bague de retenue entourant les extrémités distales desdites fibres pour retenir normalement lesdites extrémités distales en une relation côte à côte, ladite bague de retenue étant déplaçable en direction des extrémités proximales des fibres pour permettre aux fibres de s'écarter latéralement.

6. Dispositif selon la revendication 5, qui comporte en outre une bague de limitation réglable pour limiter le mouvement de ladite bague de retenue.
